# EUROPEAN PATENT APPLICATION

(11) **EP 4 477 224 A1**
(43) Date of publication of application: **18.12.2024**
(21) Application number: 23749934.8
(22) Date of filing: 30.01.2023
(51) Int. Cl.: A61K 31/519, A61K 31/426, A61P 9/00

(54) **PHARMACEUTICAL COMPOSITION COMPRISING ALLOPURINOL, FEBUXOSTAT, OR A PHARMACEUTICALLY ACCEPTABLE SALT THEREOF FOR PREVENTING OR TREATING CARDIOVASCULAR DISEASE OF SUBJECT HAVING HIGH BLOOD URIC ACID LEVEL**

(30) Priority: 07.02.2022 KR 20220015280
(71) Applicant: Indream Healthcare Inc., Jeju-si, Jeju-do 63208 (KR)
(72) Inventor: GHANG, Byeong Zu, Seogwipo-si, Jeju-do 63610 (KR)
(74) Representative: Murgitroyd & Company
(86) International application number: PCT/KR2023/001333
(87) International publication number: WO 2023/149701

(57) **Abstract**

The present invention provides a pharmaceutical composition comprising a therapeutically effective amount of allopurinol, febuxostat, or a pharmaceutically acceptable salt thereof as an active ingredient for preventing or treating cardiovascular disease, in which the composition is administered to a subject having a serum uric acid level of 6 mg/dl or higher to reduce the serum uric acid level of the subject to less than 6 mg/dl. Therefore, the pharmaceutical composition according to the present invention exhibits an excellent effect on xanthine oxidase inhibitor and is administered to selected subjects to effectively treat or prevent cardiovascular disease.

## Description

### BACKGROUND OF THE INVENTION

### Technical Field

The present disclosure relates to a pharmaceutical composition including allopurinol, febuxostat, or a pharmaceutically acceptable salt thereof, and more particularly, to a pharmaceutical composition for use in preventing or treating cardiovascular disease of a subject having a high serum uric acid concentration.

### Background Art

Cardiovascular disease is one of the leading causes of death in most countries. There have been attempts to prevent or treat the cardiovascular disease using a xanthine oxidase inhibitor, but treatment efficacy for the cardiovascular disease of the xanthine oxidase inhibitor has not been clearly revealed. Multiple studies have reported that the xanthine oxidase inhibitor rather may cause side effects related to cardiovascular disease. For example, it has been reported that when the xanthine oxidase inhibitor, in particular, febuxostat, is taken, a risk of cardiovascular disease is increased. In addition, since a method of treating cardiovascular disease by administering the xanthine oxidase inhibitor is not clinically recommended at all and there is no approval for cardiovascular disease, the treatment method has not been attempted at all.

### SUMMARY OF THE INVENTION

### Technical Goals

The present disclosure is directed to providing prevention or treatment of cardiovascular disease by administering a pharmaceutical composition including a therapeutically effective amount of allopurinol, febuxostat, or a pharmaceutically acceptable salt thereof as an active ingredient to a subject having a high serum uric acid concentration.

In particular, the present disclosure is directed to providing treatment or prevention of cardiovascular disease by administering the pharmaceutical composition according to the present disclosure to a subject selected for responding with an excellent effect when treated with allopurinol, febuxostat, or a pharmaceutically acceptable salt thereof.

### Technical solutions

According to one exemplary embodiment of the present disclosure, a pharmaceutical composition for use in preventing or treating cardiovascular disease includes a therapeutically effective amount of allopurinol, febuxostat, or a pharmaceutically acceptable salt thereof as an active ingredient. The composition is administered to a subject having a serum uric acid concentration of 6 milligrams per deciliter (mg/dl) or more to decrease the serum uric acid concentration of the subject to less than 6 mg/dl.

The pharmaceutical composition may prevent the occurrence of major cardiovascular events, and the major cardiovascular event may be death from cardiovascular disease, a myocardial infarction, a stroke, or an angina.

The subject may be 65 years of age or older.

The active ingredient may be the allopurinol or the pharmaceutically acceptable salt thereof, and the subject may have a body mass index (BMI) of 30 kilograms per square meter (kg/m²) or less.

The active ingredient may be the febuxostat or the pharmaceutically acceptable salt thereof, and the subject may have a blood glucose level of 126 mg/dl or more, a blood LDL-cholesterol level of 100 mg/dl or less, or a diastolic blood pressure of 80 millimeters of mercury (mmHg) or lower.

The subject may have a stroke or peripheral vascular disease history.

The subject may have the serum uric acid concentration of 7 mg/dl or more.

The subject may have hyperuricacidemia or gout.

The subject may have gout.

The active ingredient may be the allopurinol or the pharmaceutically acceptable salt thereof, and a dosage of the active ingredient may be 100 milligrams (mg) to 600 mg per day based on the amount of the allopurinol. For example, the dosage of the allopurinol or the pharmaceutically acceptable salt may be 100 mg, 200 mg, 300 mg, 400 mg, 500 mg, or 600 mg per day based on the amount of the allopurinol.

The active ingredient may be febuxostat or the pharmaceutically acceptable salt thereof, and the dosage of the active ingredient may be 20 mg to 120 mg per day based on the amount of the febuxostat. For example, the dosage of the febuxostat or the pharmaceutically acceptable salt may be 20 mg, 40 mg, 60 mg, 80 mg, 100 mg, or 120 mg per day based on the amount of the febuxostat.

The active ingredient may be the allopurinol or the pharmaceutically acceptable salt thereof. The active ingredient may be administered to a subject having an estimated glomerular filtration rate (eGFR) of 60 milliliters per minute per 1.73 square meters (ml/min/1.73 m²) or more at a dose of 300 mg per day based on the amount of the allopurinol, and if the serum uric acid concentration of the subject is not decreased to less than 6 mg/dl at one month after the administration, the active ingredient may be administered at a dose of 600 mg per day based on the amount of the allopurinol. The active ingredient may be administered to a subject having the eGFR of 30 ml/min/1.73 m² or more and less than 60 ml/min/1.73 m² or more at a dose of 200 mg per day based on the amount of the allopurinol, and if the serum uric acid concentration of the subject is not decreased to less than 6 mg/dl at one month after the administration, the active ingredient may be administered at a dose which increased in increments of 100 mg per month up to a maximum of 400 mg per day based on the amount of the allopurinol.

The active ingredient may be the febuxostat or the pharmaceutically acceptable salt thereof. The active ingredient may be administered at a dose of 40 mg per day based on the amount of the febuxostat, and if the serum uric acid concentration of the subject is not decreased to less than 6 mg/dl at 2 weeks after the administration, the active ingredient may be administered at a dose of 80 mg per day based on the amount of the febuxostat.

The pharmaceutical composition may further include a pharmaceutically acceptable additive or carrier.

According to another exemplary embodiment of the present disclosure, a method for preventing or treating cardiovascular disease includes administering a pharmaceutical composition including a therapeutically effective amount of allopurinol, febuxostat, or a pharmaceutically acceptable salt thereof as an active ingredient to a subject having a serum uric acid concentration of 6 mg/dl or more to decrease the serum uric acid concentration of the subject to less than 6 mg/dl.

According to another exemplary embodiment of the present disclosure, a use of a composition including a therapeutically effective amount of allopurinol, febuxostat, or a pharmaceutically acceptable salt thereof, in preparation of a drug for preventing or treating cardiovascular disease by decreasing a serum uric acid concentration of a subject having the serum uric acid concentration of 6 mg/dl or more to less than 6 mg/dl is provided.

According to still another exemplary embodiment of the present disclosure, a use of a composition including a therapeutically effective amount of allopurinol, febuxostat, or a pharmaceutically acceptable salt thereof as an active ingredient for decreasing a serum uric acid concentration of a subject having the serum uric acid concentration of 6 mg/dl or more to less than 6 mg/dl, for preventing or treating cardiovascular disease is provided.

### Effects of the Invention

According to one exemplary embodiment, cardiovascular disease may be prevented or treated by administering a pharmaceutical composition including a therapeutically effective amount of allopurinol, febuxostat, or a pharmaceutically acceptable salt thereof as an active ingredient to a subject having a high serum uric acid concentration.

In addition, according to one exemplary embodiment, cardiovascular disease may be prevented or treated more effectively by selecting a subject responding with an excellent effect when treated with a pharmaceutical composition including a therapeutically effective amount of allopurinol, febuxostat, or a pharmaceutically acceptable salt thereof as an active ingredient.

### BRIEF DESCRIPTION OF THE DRAWINGS

FIG. 1A is a graph showing the likelihood of occurrence of major cardiovascular events over time for each patient group, classified depending on the serum uric acid concentration, in an allopurinol administration group aged 65 years or older.
FIG. 1B is a graph showing the likelihood of occurrence of major cardiovascular events over time for each patient group, classified depending on the serum uric acid concentration, in an allopurinol administration group having a BMI of 30 kg/m² or less.
FIG. 2A is a graph showing the likelihood of occurrence of major cardiovascular events over time for each patient group classified depending on the serum uric acid concentration, in an febuxostat administration group aged 65 years or older.
FIG. 2B is a graph showing the likelihood of occurrence of major cardiovascular events over time for each patient group classified depending on the serum uric acid concentration, in an febuxostat administration group having a blood glucose level of 126 mg/dl or more.
FIG. 2C is a graph showing the likelihood of occurrence of major cardiovascular events over time for each patient group classified depending on the serum uric acid concentration, in an febuxostat administration group having a blood LDL-cholesterol of 100 mg/dl or less.
FIG. 2D is a graph showing the likelihood of occurrence of major cardiovascular events over time for each patient group classified depending on the serum uric acid concentration, in an febuxostat administration group having a diastolic blood pressure of 80 mmHg or lower.
FIG. 2E is a graph showing the likelihood of occurrence of major cardiovascular events over time for each patient group classified depending on the serum uric acid concentration, in an febuxostat administration group having a stroke history.
FIG. 2F is a graph showing the likelihood of occurrence of major cardiovascular events over time for each patient group classified depending on the serum uric acid concentration, in an febuxostat administration group having a peripheral vascular disease history.

### DETAILED DESCRIPTION OF THE EMBODIMENTS

Unless otherwise defined, all technical and scientific terms used in the present specification have the same meaning as commonly understood by a person skilled in the art to which the present disclosure pertains. In general, the terminology used in the present specification is well-known and commonly used in the art.

The term "and/or" in the present specification is used as a term referring to combinations of all configurations derived from a plurality of constituent elements.

According to one exemplary embodiment of the present disclosure, a pharmaceutical composition for use in preventing or treating cardiovascular disease including a therapeutically effective amount of allopurinol, febuxostat, or a pharmaceutically acceptable salt thereof as an active ingredient is provided. In the present specification, a pharmaceutically acceptable salt thereof refers to a pharmaceutically acceptable salt of allopurinol or a pharmaceutically acceptable salt of febuxostat.

Allopurinol and febuxostat are drugs used for hyperuricacidemia patients, and since they may inhibit a xanthine oxidase, they inhibit conversion of hypoxanthine or xanthine into uric acid, and thus, decrease a serum uric acid concentration and decrease deposition of uric acid in, for example, bone and joints.

Structures of the allopurinol and the febuxostat are as follows:
[Allopurinol] 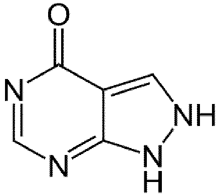
[Febuxostat]

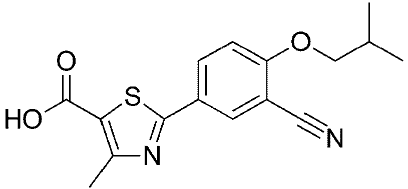

Allopurinol and febuxostat are used for preventing or treating hyperuricacidemia or gout, but the therapeutic effectiveness for cardiovascular disease has not been clearly revealed so far, and rather, multiple studies are being reported that they make cardiovascular disease worse, and thus, a method for treating cardiovascular disease using allopurinol and febuxostat is not clinically attempted at all.

Therefore, it may be an important task to confirm a cardiovascular disease patient group who responds with an effective prevention or treatment effect by administering a xanthine oxidase inhibitor such as allopurinol and febuxostat. That is, it is important to select a patient group who respond with a particularly excellent effect when treated with a xanthine oxidase inhibitor such as allopurinol and febuxostat to selectively treat the subjects who has an effect from allopurinol and febuxostat.

Cardiovascular disease (CVD) refers to a disease related to heart or blood vessels. Cardiovascular disease may include coronary heart disease, coronary vascular disease, arteriosclerosis, hypertension, angina pectoris, myocardial infarction, acute renal failure, congestive cardiac failure, peripheral vascular disease, heart attack, arrhythmia, tachycardia, stroke, transient ischemic attack, and cerebrovascular disease. Prevalence of cardiovascular disease is increasing due to aging, and the cardiovascular disease is the leading cause of death worldwide.

In the present disclosure, a subject may refer to a mammal including a human, and for example, the subject may be a human.

In the present disclosure, "treatment" refers to reverse, alleviation, inhibition of progression of, or prevention of a disease or illness, or one or more symptoms of the disease or illness, unless otherwise stated, and the term "treatment" used in the present disclosure refers to the act of treating when the "treatment" is defined as described above. Therefore, the composition and the method of the present disclosure may be used for, for example, chronic treatment as well as treatment as a therapeutic treatment for a certain period of time.

In the present specification, the term "prevention" may refer to prevention or delay of onset of disease, disorder, or illness.

The composition of the present disclosure may be formulated into various forms, for example, oral formulations such as liquids, suspensions, powders, granules, tablets, capsules, pills, extracts, emulsions, syrups, and aerosols, and injection of a sterile injection solution according to a common method for each intended use and then used, and may be orally administered or parenterally administered through various routes including intravenous, intraperitoneal, subcutaneous, intradermal, intramuscular, spinal, intrathecal, or rectal topical administration or injection, and a dosage ranges may vary depending on, for example, a patient's weight, age, sex, health status, diet, administration time, administration method, administration period or interval, excretion rate, and constitutional specificity, the nature of the preparation, and the severity of disease. For example, the composition of the present disclosure may be an oral dosage form in a tablet form. Otherwise, the composition of the present disclosure may be an intravenous injection form in a liquid form.

The term "tablet" in the present specification refers to a medicine compressed into a small disc shape for easy taking. The tablet may include, for example, an uncoated tablet, a film-coated tablet, a sugar-coated tab let, a multilayer tab let, a cored tablet, an inner-core tablet, an orally disintegrating tablet, a chewable tab let, an effervescent tablet, a dispersible tablet, and a dissolving tablet.

In one exemplary embodiment, the composition of the present disclosure may be a composition for oral administration. The term "oral administration" in the present specification may refer to administration of an active material to a gastrointestinal tract by an oral route for absorption. A non-limiting example of the preparation for oral administration may include, for example, tablets, troches, lozenges, aqueous suspensions, oily suspensions, prepared powders, granules, emulsions, hard capsules, soft capsules, syrups, or elixirs. In order to prepare the pharmaceutical composition of the present disclosure into a formulation for oral administration, a pharmaceutically acceptable additive or carrier may be further included. For example, the pharmaceutical composition of the present disclosure may use, for example, binders such as lactose, saccharose, sorbitol, mannitol, starch, amylopectin, cellulose, or gelatin; excipients such as dicalcium phosphate; disintegrants such as corn starch or sweet potato starch; and lubricants such as magnesium stearate, calcium stearate, sodium stearyl fumarate, or polyethylene glycol, and also, for example, sweeteners, flavorings, or syrups. Furthermore, the capsule may further use, for example, a liquid carrier such as fatty oil in addition to the materials mentioned above.

The composition of the present disclosure may be a composition for parenteral administration. The term "parenteral administration" in the present specification refers to intravenous, intraperitoneal, subcutaneous, intradermal, intramuscular, spine, intrathecal, or rectal topical administration or injection. The parenteral administration depends on a method of injecting suppository, subcutaneous injection, intramuscular injection, or intrathoracic injection. Herein, the composition is mixed with a stabilizer or buffer in water for preparing into a formulation for parenteral administration and prepared into a solution or suspension, which may be prepared into a unit dosage form of an ampoule or vial.

The pharmaceutical composition according to one exemplary embodiment of the present disclosure may further include other active ingredients in addition to the allopurinol, the febuxostat, or the pharmaceutically acceptable salt thereof. For example, the pharmaceutical composition according to one exemplary embodiment may further include other therapeutic agents for kidney disease. For example, the pharmaceutical composition according to one exemplary embodiment may further include one or two or more of active ingredients such as an angiotensin converting enzyme inhibitor (ACE I) or an angiotensin II receptor blocker (ARB). The pharmaceutical composition according to one exemplary embodiment of the present disclosure may be, for example, a composition for administration in combination.

In the present disclosure, the term "pharmaceutically acceptable salt" refers to a salt commonly used in the pharmaceutical industry. For example, it may refer to an acid addition salt formed by a pharmaceutically acceptable free acid. For example, a non-limiting example of the pharmaceutically acceptable salt may include inorganic ion salts prepared by, for example, calcium, potassium, sodium, or magnesium, or inorganic acid salts prepared by, for example, hydrochloric acid, nitric acid, bromic acid, iodic acid, perchloric acid, or sulfuric acid; organic acid salts prepared by, for example, acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid, or vanillic acid; sulfonic acid salts prepared by, for example, methanesulfonic acid, ethanesulfonic acid, benzenesulfonic acid, p-toluenesulfonic acid, or naphthalene sulfonic acid; amino acid salts prepared by glycine, arginine, or lysine; or amine salts prepared by, for example, trimethylamine, triethyleamine, ammonia, pyridine, or picoline.

According to one exemplary embodiment of the present disclosure, a pharmaceutical composition for use in preventing or treating cardiovascular disease of a subject having a serum uric acid concentration of 6 mg/dl or more, including a therapeutically effective amount of allopurinol, febuxostat, or a pharmaceutically acceptable salt thereof as an active ingredient may be provided.

In one exemplary embodiment, the pharmaceutical composition prevents occurrence of major cardiovascular events, and the major cardiovascular event may be death from cardiovascular disease, a myocardial infarction, a stroke, or an angina. The myocardial infarction may include fatal or nonfatal myocardial infarction, and the stroke may include fatal or nonfatal stroke. The angina may include angina with coronary revascularization, and may be, for example, unstable angina with coronary revascularization.

In one exemplary embodiment, the subject may be 65 years of age or older.

In one exemplary embodiment, the active ingredient may be the allopurinol or the pharmaceutically acceptable salt thereof, and the subject may have a BMI of 30 kg/m² or less.

In the present specification, BMI refers to a value obtained by dividing the weight of the subject by the square of the height of the subject.

In one exemplary embodiment, the active ingredient may be the febuxostat or the pharmaceutically acceptable salt thereof, and the subject may have a blood glucose level of 126 mg/dl or more, a blood LDL-cholesterol level of 100 mg/dl or less, and/or a diastolic blood pressure of 80 mmHg or lower.

In one exemplary embodiment, the subject may have a stroke or peripheral vascular disease history.

In one exemplary embodiment, the subject may have the serum uric acid concentration of 7 mg/dl or more. The subject may be a patient with hyperuricacidemia or gout who has the serum uric acid concentration of 7 mg/dl or more.

In one exemplary embodiment, the subject may have hyperuricacidemia or gout. In one exemplary embodiment, the subject may have gout.

In one exemplary embodiment, the composition may decrease the serum uric acid concentration of the subject to less than 6 mg/dl. The composition of one exemplary embodiment maintains the serum uric acid concentration of the subject at less than 6 mg/dl to prevent or treat the chronic kidney disease. In particular, the pharmaceutical composition of one exemplary embodiment may show an excellent effect in both prevention and treatment of cardiovascular disease in that it prevents or reduces occurrence of major cardiovascular events in the subject, and reduces or alleviates occurrence of major cardiovascular events in patients having a cardiovascular disease history.

In one exemplary embodiment, the active ingredient may be the allopurinol or the pharmaceutically acceptable salt thereof, and a dosage of the active ingredient may be 100 mg to 600 mg, 300 mg to 600 mg, or 200 mg to 400 mg per day, and for example, 100 mg, 200 mg, 300 mg, 400 mg, 500 mg, or 600 mg per day, based on the amount of the allopurinol.

In one exemplary embodiment, the active ingredient may be the febuxostat or the pharmaceutically acceptable salt thereof, and the dosage of the active ingredient may be 20 mg to 120 mg, 20 mg to 100 mg, or 40 mg to 80 mg per day, for example, 20 mg, 40 mg, 60 mg, 80 mg, 100 mg, or 120 mg per day, based on the amount of the febuxostat.

The daily dosage in the present disclosure may be a dosage administered 1 to 5 times per day, for example, a dosage administered once a day, twice a day, or 3 times a day, and in particular, a dosage administered once a day.

Unless otherwise mentioned in the present disclosure, the dosage or content of the pharmaceutically acceptable salt of the active ingredient may be based on the amount of the active ingredient excluding salts. That is, when it is mentioned in the present specification that the dosage or content of the pharmaceutically acceptable salt of the allopurinol is 200 mg, it is interpreted as meaning 200 mg of allopurinol excluding salts.

In one exemplary embodiment, the active ingredient may be the allopurinol or the pharmaceutically acceptable salt thereof. The allopurinol or the pharmaceutically acceptable salt may be administered to the subject having the eGFR of 60 ml/min/1.73 m² or more at a dose of 300 mg per day, and if the serum uric acid concentration of the subject is not decreased to less than 6 mg/dl at one month after the administration, the active ingredient may be administered at a dose of 600 mg per day.

The allopurinol or the pharmaceutically acceptable salt thereof may be administered to the subject having the eGFR of 30 ml/min/1.73 m² or more to less than 60 mg/min/1.73 m² at a dose of 200 mg per day, and if the serum uric acid concentration of the subject is not decreased to less than 6 mg/dl at one month after the administration, may be administered at a dose which increased in increments of 100 mg per month up to a maximum of 400 mg per day.

In one exemplary embodiment, the active ingredient may be the febuxostat or the pharmaceutically acceptable salt thereof. The febuxostat or the pharmaceutically acceptable salt thereof may be administered at a dose of 40 mg per day based on the amount of the febuxostat, and if the serum uric acid concentration of the subject is not decreased to less than 6 mg/dl 2 weeks after the administration, may be administered at a dose of 80 mg per day based on the amount of the febuxostat.

The pharmaceutical composition of one exemplary embodiment may be administered to a patient with a major cardiovascular disease history. The major cardiovascular disease history may be at least one selected from hospitalization for myocardial infarction or unstable angina, hospitalization for stroke or transient ischemic attack, peripheral vascular disease and diabetes mellitus symptoms.

The pharmaceutical composition of one exemplary embodiment may be a composition in an oral dosage form. The pharmaceutical composition of one exemplary embodiment may be a tablet in an oral dosage form. The pharmaceutical composition of one exemplary embodiment may be an intravenous dosage form.

Hereinafter, the present disclosure will be described in more detail based on the following examples. These examples are provided merely to illustrate the present disclosure, and it will be apparent to a person skilled in the art that the scope of the present disclosure is not construed to be limited to the examples.

### Examples

### Design of clinical trial

In order to evaluate non-inferiority of febuxostat to allopurinol related to cardiovascular risk, a clinical trial was designed and proceeded.

### Trial design

A multicenter, randomized, double-blind non-inferiority trial was performed. The details of the clinical trial design have been published previously. An independent data and safety monitoring committee with access to unblinded data monitored the clinical trial. The statistical analysis for the data and the safety monitoring committee was performed by an independent statistical group (WebbWrites).

### Patient group

Patients who were diagnosed with gout fulfilling category 9 of the standards set by the American Rheumatism Association had a history of major cardiovascular diseases before randomization were enrolled as participants in the clinical trial. As an additional criterion for clinical trial participation, patients with inappropriately controlled gout who had a serum uric acid concentration of at least 7.0 mg per deciliter (420 µmol per liter) or more or 6.0 mg per deciliter (360 µmol) or more after a 1 to 3 weeks washout period from the previous gout therapies were required. The patients were regarded as having a history of major cardiovascular diseases, if they had myocardial infarction, hospitalization for unstable angina, stroke, hospitalization for transient ischemic attack, peripheral vascular disease, or diabetes mellitus symptoms. All participants provided a prior informed consent form.

### Treatments and procedures

The clinical trial participants participated in the clinical trial according to the following procedures. Hereinafter, unless otherwise particularly mentioned, a dose of a drug was described based on an amount of allopurinol or febuxostat.

### (1) Allopurinol administration group

The patients were randomly assigned so that they received febuxostat or allopurinol administered once a day in a double-blind manner. The randomization was stratified according to the estimated creatinine clearance at baseline (patient group having an estimated creatinine clearance ≥60 ml per minute vs patient group having an estimated creatinine clearance ≥30 and <60 ml per minute).

The dose of allopurinol was modified according to a kidney function. The patients with the estimated creatinine clearance 60 ml or more per minute i) initially received allopurinol at a dose of 300 mg once a day, and the dose was increased in 100 mg increments monthly until the patient had the serum uric acid concentration of less than 6.0 mg per deciliter or the dosage of allopurinol was 600 mg once a day. The patient having the estimated creatinine clearance of 30 ml or more and less than 60 ml per minute initially received 200 mg of allopurinol, and then the dose was increased in 100 mg increments until the patient either had the serum uric acid concentration of less than 6.0 mg per deciliter or was receiving the allopurinol dose of 400 mg once daily.

### (2) Febuxostat administration group

The dose of febuxostat was not modified according to the kidney function. The patients who were randomly assigned to receive febuxostat initially received 40 mg once a daily, and continued to receive the same dosage if the serum uric acid concentration was less than 6.0 mg per deciliter after 2 weeks of the treatment. If the serum uric acid concentration was higher than 6.0 mg per deciliter at 2nd week visit, the dose of febuxostat was increased to 80 mg once daily for the remainder of the trial.

### (3) Procedure

The serum uric acid concentrations of the patients were revealed to the site investigators only during the dose adjustment period of 10 weeks to facilitate dose increases based on a uric acid response. During the period, the administration of a double-blind and double-dummy trial medications was guided by an interactive voice-response system which was a procedure to prevent unblinding for the patients whose dose was not adjusted. After completing dose adjustment, the urate level was concealed from the investigators, and the treatment was managed throughout the trial using the interactive voice-response system.

At the screening visit, all uric acid lowering therapy was discontinued, and a colchicine treatment at a dose of 0.6 mg per day was started for preventing a gout flare unless the patient had a history of unacceptable side effects due to colchicine. All patients received prophylaxis for the first 6 months of the randomly assigned treatment. If the colchicine treatment resulted in unacceptable side effects and the estimated creatinine clearance was at least 50 ml per minute, the patient received naproxen (250 mg, twice a day) with lansoprazole (15 mg, once a day). If the patient did not receive colchicine or naproxen, other nonsteroidal anti-inflammatory drugs (NSAID) or prednisone could be provided as prophylaxis, or the investigators could choose to manage a gout flare, if it occurred.

Outpatient visits were conducted according to the following schedule: during screening and randomization, and at 2, 4, 6, 8, 10, 12, and 24 weeks after the randomization, and every 6 months during the subsequent years of the trial. The patients with reduced kidney function or, the patients aged 65 years or older at randomization were visited at 9 and 15 months for monitoring the serum chemical profile. If the patient agreed to be monitored, but would not return for study visit, telephone contact was made, but such method was not preferred or recommended.

### End point

A first occurrence of cardiovascular death, nonfatal myocardial infarction, nonfatal stroke, or urgent revascularization for unstable angina were set as a primary composite end point.

A secondary safety end point included a composite of cardiovascular death, nonfatal myocardial infarction, and nonfatal stroke, and the individual components of the primary evaluation variables. The consistency of the effects on the primary end point was investigated in various subgroups (both pre-specified and post-hoc designation). An additional safety end point included death from all causes, urgent cerebral revascularization, transient ischemic attack, hospitalization for cardiac failure, arrhythmias not associated with ischemia, and venous thromboembolic events. An independent central end point committee consisting of members who did not know about the treatment assignment adjudicated all suspected end point events.

### Results

### Patient group

6198 patients from 320 locations across North America enrolled from April 2010 to May 2017. 8 patients did not receive the trial medication, and 6190 patients received modified intention to treat analysis. Two treatment groups were confirmed to be well balanced with regard to all baseline characteristics. In a febuxostat group, 61.0% of the patients received 40 mg of febuxostat every day, and 39.0% of the patients received 80 mg of febuxostat every day as a final adjusted dose. In an allopurinol group, 21.8% of the patients received 200 mg of allopurinol, 44.6% of the patients received 300 mg of allopurinol, 25.2% of the patients received 400 mg of allopurinol, 4.3% of the patients received 500 mg of allopurinol, and 4.1% of the patients received 600 mg of allopurinol, on the basis of the protocol-directed criteria for the estimated creatinine clearance.

Overall, 56.6% of the patients discontinued the trial treatment prematurely. The early discontinuation rates were similar in the febuxostat and allopurinol groups (57.3% in the febuxostat group and 55.9% in the allopurinol group). A percentage of the patients who did not complete all trial visits was 45.0% (45.0% in the febuxostat, 44.9% in the allopurinol group). The median duration of exposure to febuxostat was 728 days, and the median duration of exposure to allopurinol was 719 days. The median duration of a follow-up period was 968 days in the febuxostat group and 942 days in the allopurinol group.

### Biochemical effect

A proportion of the patients with the serum uric acid concentration of less than 6.0 mg per deciliter was higher in the febuxostat group than in the allopurinol group at the 2nd week. Thereafter, though a difference between the groups was not large, a higher proportion of the patients in the febuxostat group had maintenance of the serum uric acid concentration at less than 6.0 mg per deciliter at most times. In addition, a larger proportion of the patients in the febuxostat group than in the allopurinol group had the serum uric acid concentration of less than 5.0 mg per deciliter (300 µmol per liter) for the entire trial. Overall, the rates of the gout flare were similar in the two treatment groups (flares of 0.68 and 0.63, per patient-year in the febuxostat group and the allopurinol group, respectively). During the trial period, there was no significant difference in serum levels of electrolyte, glucose, or lipids, or blood pressure, and also, there was no difference in use of cardiovascular medications.

### Analysis of effect on cardiovascular patient for each patient group

In order to confirm the effect of allopurinol and febuxostat on the cardiovascular disease for each patient group, the data obtained in the clinical trial described above was analyzed.

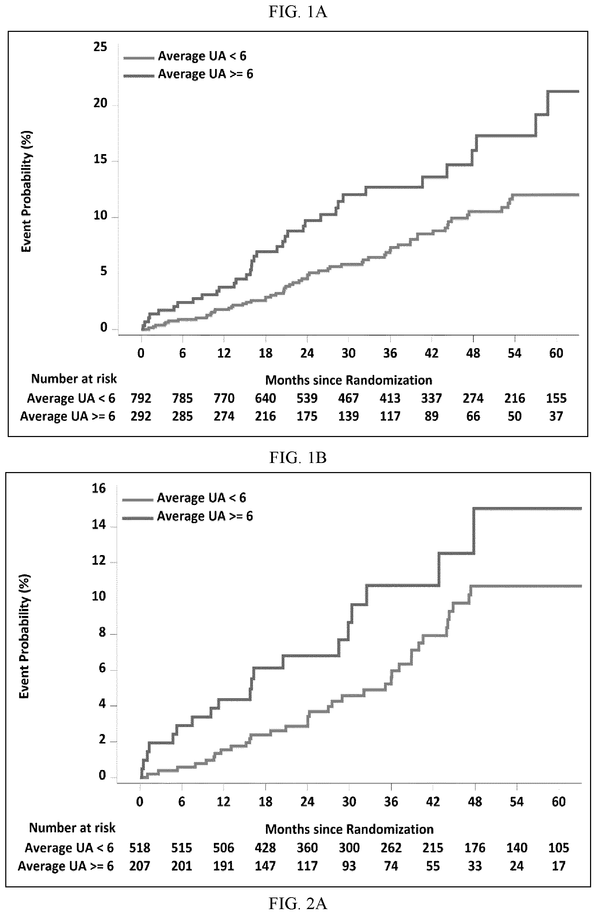

The patients were classified into patient groups according to specific biomarkers such as age, blood glucose level, blood LDL-cholesterol level, blood pressure, and history of certain diseases, and then risk of developing or worsening the cardiovascular disease in patients who had the serum uric acid concentration decreased to less than 6 and patients who maintained the serum uric acid concentration at or above 6 after the administration of allopurinol and febuxostat were confirmed using a hazard ratio (HR), and the results are shown in Tables 1 and 2. In addition, specific data are shown in FIGS. 1A, 1B, and 2A to 2F. The hazard ratio is a value obtained by dividing the risk of an experimental group by the risk of a control group, and if the hazard ratio is 1, it means that the risk of the experimental group and the risk of the control group are the same, and if the hazard ratio is 2, it means that the risk rate of the control group is twice the risk rate of the experimental group.

In Tables 1 and 2, a confidence interval is indicated as CI, and diastolic blood pressure is indicated as DBP.

The risk of occurrence of major cardiovascular events of the patients who had the serum uric acid concentration decreased to less than 6 and the patients who had maintenance of the serum uric acid concentration at or above 6 by administering allopurinol was confirmed, and the results are shown in the following Table 1.

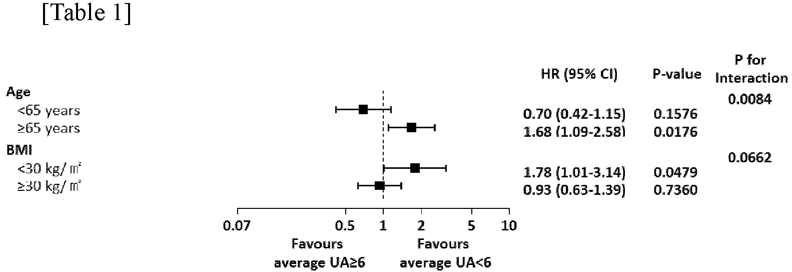

Referring to Table 1, it was confirmed that if the serum uric acid concentration was maintained at or above 6 in the patient group aged 65 years or older, the risk of occurrence of the major cardiovascular events was increased by about 1.68 times, and if the serum uric acid concentration was maintained at or above 6 in the patent group having a BMI of 30 kg/m² or less, the risk of occurrence of the major cardiovascular events was increased by about 1.78 times.

More detailed data are shown in FIGS. 1A and 1B. FIG. 1A is a graph showing the likelihood of occurrence of major cardiovascular events over time for each patient group, classified depending on the serum uric acid concentration, in an allopurinol administration group aged 65 years or older. In FIG. 1A, a p value measured by a log rank test was 0.0021. FIG. 1B is a graph showing the likelihood of occurrence of major cardiovascular events over time for each patient group, classified depending on the serum uric acid concentration, in an allopurinol administration group having a BMI of 30 kg/m² or less. In FIG. 1B, a p value measured by a log rank test was 0.0851.

Referring to FIGS. 1A and 1B, it was confirmed that if each patient group classified based on both age and BMI maintained the serum uric acid concentration below 6, the likelihood of occurrence of major cardiovascular events was considerably decreased as compared with the patient group having the serum uric acid concentration of 6 or more.

That is, it was confirmed that if allopurinol is administered to a patient group aged 65 years or older or having a BMI of 30 kg/m² or less to maintain the serum uric acid concentration below 6, the cardiovascular disease may be effectively prevented or treated.

In Table 2, the risk of occurrence of the major cardiovascular events of the patients who had the serum uric acid concentration decreased to less than 6 and the patients who maintained the serum uric acid concentration at or above 6 by administering febuxostat are confirmed using the hazard ratio.

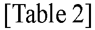

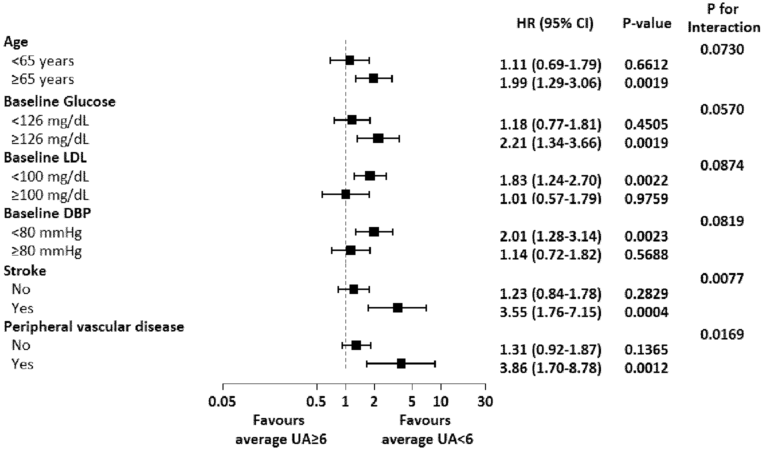

Referring to Table 2, it is confirmed that if febuxostat is administered to a patient group aged 65 years or older, or having a blood glucose level of 126 mg/dl or more, a blood LDL-cholesterol level of 100 mg/dl or less, a diastolic blood pressure of 80 mm/Hg or lower, or a stroke or peripheral vascular disease history to maintain the serum uric acid concentration below 6, the cardiovascular disease may be prevented or treated.

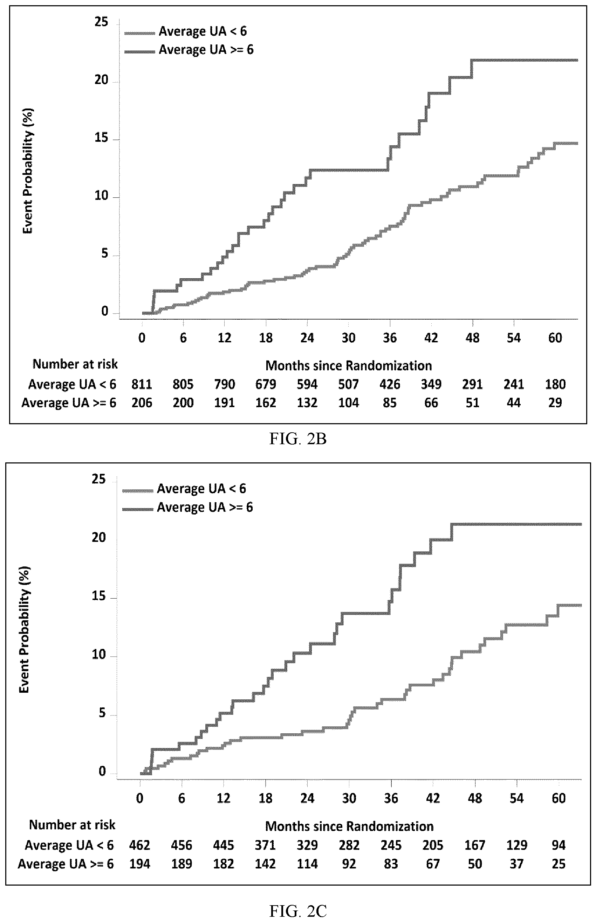

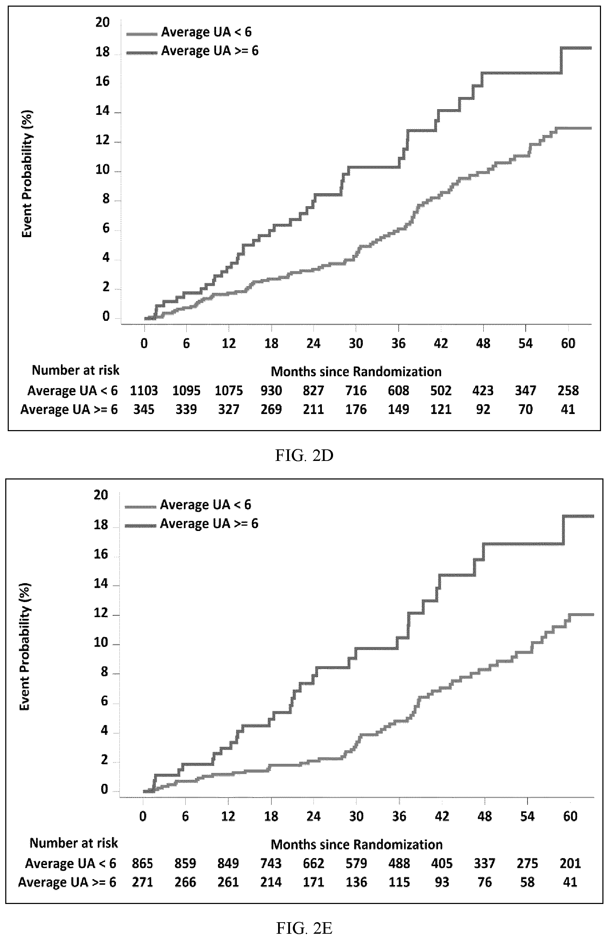

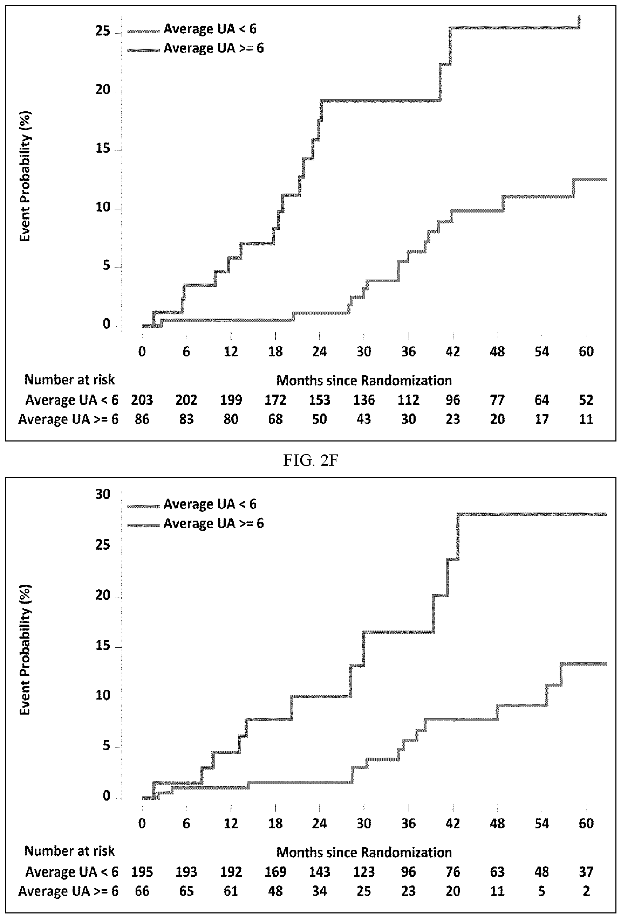

Detailed data are shown in FIGS. 2A to 2F. FIG. 2A is a graph showing the likelihood of occurrence of major cardiovascular events over time for each patient group classified depending on the serum uric acid concentration, in an febuxostat administration group aged 65 years or older. In FIG. 2A, a p value measured by a log rank test was 0.0006. FIG. 2B is a graph showing the likelihood of occurrence of major cardiovascular events over time for each patient group classified depending on the serum uric acid concentration, in an febuxostat administration group having a blood glucose level of 126 mg/dl or more. In FIG. 2B, a p value measured by a log rank test was 0.0017. FIG. 2C is a graph showing the likelihood of occurrence of major cardiovascular events over time for each patient group classified depending on the serum uric acid concentration, in an febuxostat administration group having a blood LDL-cholesterol level of 100 mg/dl or less. In FIG. 2C, a p value measured by a log rank test was 0.0026. FIG. 2D is a graph showing the likelihood of occurrence of major cardiovascular events over time for each patient group classified depending on the serum uric acid concentration, in an febuxostat administration group having a diastolic blood pressure of 80 mmHg or lower. In FIG. 2D, a p value measured by a log rank test was 0.0003. FIG. 2E is a graph showing the likelihood of occurrence of major cardiovascular events over time for each patient group classified depending on the serum uric acid concentration, in an febuxostat administration group having a stroke history. In FIG. 2E, a p value measured by a log rank test was 0.0002. FIG. 2F is a graph showing the likelihood of occurrence of major cardiovascular events over time for each patient group classified depending on the serum uric acid concentration, in an febuxostat administration group having a peripheral vascular disease history. In FIG. 2F, a p value measured by a log rank test was 0.0014.

Referring to FIGS. 2A to 2F, it was confirmed that if all patient groups classified based on every aforementioned biomarkers maintained the serum uric acid concentration below 6, the likelihood of occurrence of major cardiovascular events was considerably decreased as compared with the patient group having the serum uric acid concentration of 6 or more.

From the above results, it was confirmed that if the pharmaceutical composition according to embodiments described herein is administered to a subject with a serum uric acid concentration of 6 ml/dl or more, a subject aged 65 years or older, a subject with a BMI of 30 kg/m² or less, a subject with a blood glucose level of 126 mg/dl or more, a subject with a blood LDL-cholesterol level of 100 mg/dl or less, a subject with a diastolic blood pressure of 80 mmHg or lower, or a subject with a stroke or peripheral vascular disease history, to maintain the serum uric acid concentration below 6 ml/dl, a remarkably excellent effect may be shown in prevention or treatment of cardiovascular disease.

According to the present disclosure, a pharmaceutical composition is provided that may prevent or treat cardiovascular disease more effectively by selecting a subject group who may respond with an excellent effect when administered.

## Claims

1. A pharmaceutical composition for use in preventing or treating cardiovascular disease comprising: a therapeutically effective amount of allopurinol, febuxostat, or a pharmaceutically acceptable salt thereof as an active ingredient,
wherein the composition is administered to a subject having a serum uric acid concentration of 6 mg/dl or more to decrease the serum uric acid concentration of the subject to less than 6 mg/dl.

2. The pharmaceutical composition for use according to claim 1, wherein the pharmaceutical composition prevents occurrence of a major cardiovascular event, and wherein the major cardiovascular event is death from cardiovascular disease, a myocardial infarction, a stroke, or an angina.

3. The pharmaceutical composition for use according to claim 1, wherein the subject is 65 years of age or older.

4. The pharmaceutical composition for use according to claim 1, wherein the active ingredient is the allopurinol or the pharmaceutically acceptable salt thereof, and wherein the subject has a body mass index (BMI) of 30 kg/m² or less.

5. The pharmaceutical composition for use according to claim 1, wherein the active ingredient is the febuxostat or the pharmaceutically acceptable salt thereof, and wherein the subject has a blood glucose level of 126 mg/dl or more, a blood LDL-cholesterol level of 100 mg/dl or less, or a diastolic blood pressure of 80 mmHg or lower.

6. The pharmaceutical composition for use according to claim 1, wherein the subject has a stroke or peripheral vascular disease history.

7. The pharmaceutical composition for use according to claim 1, wherein the subject has the serum uric acid concentration of 7 mg/dl or more.

8. The pharmaceutical composition for use according to claim 1, wherein the subject has hyperuricacidemia or gout.

9. The pharmaceutical composition for use according to claim 1, wherein the subject has gout.

10. The pharmaceutical composition for use according to claim 1, wherein the active ingredient is the allopurinol or the pharmaceutically acceptable salt thereof, and wherein a dosage of the active ingredient is 100 mg to 600 mg per day based on an amount of the allopurinol.

11. The pharmaceutical composition for use according to claim 1, wherein the active ingredient is the febuxostat or the pharmaceutically acceptable salt thereof, and wherein a dosage of the active ingredient is 20 mg to 120 mg per day based on an amount of the febuxostat.

12. The pharmaceutical composition for use according to claim 1,
wherein the active ingredient is the allopurinol or the pharmaceutically acceptable salt thereof,
wherein the active ingredient is administered to the subject having an estimated glomerular filtration rate (eGFR) of 60 ml/min/1.73 m² or more at a dose of 300 mg per day based on an amount of the allopurinol, and if the serum uric acid concentration of the subject is not decreased to less than 6 mg/dl at one month after the administration, the active ingredient is administered at a dose of 600 mg per day based on the amount of the allopurinol, and
wherein the active ingredient is administered to the subject having the eGFR of 30 ml/min/1.73 m² or more and less than 60 ml/min/1.73 m² at a dose of 200 mg per day based on the amount of the allopurinol, and if the serum uric acid concentration of the subject is not decreased to less than 6 mg/dl at one month after the administration, the active ingredient is administered at a dose which increased in increments of 100 mg per month up to a maximum of 400 mg per day based on the amount of the allopurinol.

13. The pharmaceutical composition for use according to claim 1, wherein
the active ingredient is the febuxostat or the pharmaceutically acceptable salt thereof, and
wherein the active ingredient is administered at a dose of 40 mg per day based on an amount of the febuxostat, and if the serum uric acid concentration of the subject is not decreased to less than 6 mg/dl at 2 weeks after the administration, the active ingredient is administered at a dose of 80 mg per day based on the amount of the febuxostat.
